(19)

Europäisches Patentamt
European Patent Office
Office européen des brevets

(11) **EP 4 095 528 A1**

(12) **EUROPEAN PATENT APPLICATION**

(43) Date of publication:
**30.11.2022 Bulletin 2022/48**

(21) Application number: **21176724.9**

(22) Date of filing: **28.05.2021**

(51) International Patent Classification (IPC):
**G01N 30/96** (2006.01)　**G01N 30/88** (2006.01)
**G01N 33/18** (2006.01)　G01N 21/33 (2006.01)
**G01N 30/74** (2006.01)　H01L 25/16 (2006.01)

(52) Cooperative Patent Classification (CPC):
**G01N 30/74; G01N 30/88;** G01N 30/78;
G01N 33/182; G01N 33/188; G01N 2030/0095

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **AQUAMONITRIX LIMITED**
**Tullow, Carlow (IE)**

(72) Inventors:
• **Murray, Eoin**
**Tullow, R93 N529 (IE)**

• **Fitzhenry, Cian**
**Tullow, R93 N529 (IE)**
• **Jowett, Liam**
**Tullow, R93 N529 (IE)**
• **Roche, Patrick**
**Tullow, R93 N529 (IE)**

(74) Representative: **Murgitroyd & Company**
**Murgitroyd House**
**165-169 Scotland Street**
**Glasgow G5 8PL (GB)**

(54) **PORTABLE ANALYSER USING TWO-DIMENSIONAL ION CHROMATOGRAPHY FOR THE DETECTION OF INORGANIC ANALYTES IN WATER SAMPLES WITH DIFFERENT LEVELS OF SALINITY**

(57)　The present invention provides a portable in situ Ion Chromatography system (100) for detecting inorganic analytes in water samples obtained from freshwater and marine aquatic environments. The system (100) of the present invention is provided with an optical detection system (110), which is configured to be dynamically operated, based on the salinity level of the water-based sample, between a marine analysis mode for analysing water-based samples having a salinity level within a first threshold value range, where in the marine analysis mode the second optical analyser module is activated such that the water-based sample exiting the first optical analyser module is directed for further analysis to the second optical analyser module, and a freshwater analysis mode for analysing water-based samples having a salinity level within a second threshold value range, where in the second analysis mode the second optical analyser is deactivated and the water-based sample exiting the first optical analyser module is directed to waste.

Figure 2

**Description**

**Field**

The present invention relates to a portable system and a method for detecting inorganic analytes such as nitrate and nitrite in a water-based sample. In particular, the present invention relates to a portable system for detecting inorganic analytes in freshwater and seawater.

**Background**

Real-time or near real-time, in-situ portable analysers are paving the way for future monitoring and understanding of environmental waters. These analysers can generate accurate, continuous, and repeatable quantification of inorganic analytes such as nitrate, which occurs naturally in seawater and freshwater ecosystem.

Systems capable of generating accurate, continuous, and repeatable quantification of nutrients such as nitrate are of particular appeal, with portability and robustness being of paramount importance for in-situ water analysis.

An example of a recent analyser is disclosed in WO2019228686A1. The analyser is based on rapid ion chromatography (IC) and direct UV detection using a 235 nm LED and photodiode. By coupling the UV LED-based detection with rapid ion separation, selective detection of both nitrite and nitrate was achieved. The analyser was deployed in a range of environmental and industrial waters and demonstrated accuracy and precision comparable to laboratory-based instrumentation. However, this IC-based analyser is not applicable to marine analysis due to the sample complexity associated with saline samples, such as brackish waters typical of estuaries or coastal waters.

The determination of nitrate and other inorganic anions in water samples with high salt content is a major challenge for IC and is demanding due to the deleterious effects which the high levels of chloride and other anions have on the anion exchange separation.

Current on-column matrix elimination approaches for mitigating the matrix effects of challenging samples, such as seawater, use the major matrix ion as the eluent. For example, as reported by Haddad et al., "On-column matrix elimination of high levels of chloride and sulfate in non-suppressed ion chromatography" J. Chromatogr. A. 546 (1991) 221-228, on-column matrix elimination using NaCl eluent in which the chloride matrix ion in the eluent overcame the matrix effect of saline samples and allowed for the detection of various anions, including nitrate, using IC with direct UV detection.

Furthermore, Ito et al., "Determination of nitrite, nitrate, bromide, and iodide in seawater by ion chromatography with UV detection using dilauryldimethylammonium-coated monolithic ODS columns and sodium chloride as an eluent" Analytical and Bioanalytical Chemistry. 404 (2012) 2513-2517 discloses matrix elimination using dilauryldimethylammonium-coated monolithic ODS columns and direct UV detection.

Wang et al., "Determination of low-level anions in seawater by ion chromatography with cycling-column-switching" J. Chromatogr. A 1265 (2012) 186-190 describes an alternative approach to overcome the matrix effects of seawater using a cycling-column-switching approach. Within this system, low concentration anions were concentrated before final analysis, removing the effect of the matrix ion.

Two-dimensional (2D) ion chromatography has emerged as a promising technique for the analysis of trace ions, particularly in challenging matrices. However, limitations still exist when considering cost-effective, in-situ detection of inorganic analytes in both marine and natural waters.

There is a need to provide a portable system that can be deployed directly in the desired monitoring environment and configured to detect inorganic analytes in water samples having different salinity levels.

**Summary**

The aim of the present invention is to provide a portable in situ Ion Chromatography system and method for detecting inorganic analytes in a water-based sample having different levels of salinity. More, in particular, it is an object of the present application to provide a portable in situ Ion Chromatography system configured to detect inorganic analytes in water samples having different levels of salinity, especially by avoiding interferences and coelution associated with the high anion concentrations present within the seawater matrix.

According to a first aspect of the present invention, a portable in-situ Ion Chromatography system is provided for detecting inorganic analytes in water-based samples having different levels of salinity, the system comprising:

a housing defining an internal space and comprising a cover configured to removably cover the internal space;
an optical detection system disposed within the internal space and configured to receive and analyse awater-based sample for the detection of inorganic analytes, the optical detection system comprising

a first optical analyser module configured to receive and analyse the water-based sample;
a second optical analyser module configured, when activated, to receive and analyse the water-based sample exiting the first optical analyser module; and
a processing unit configured to determine the levels of inorganic analytes in the water-based sample based on information received from the first and/or second optical analysers;

wherein the processing unit is configured to operate, based on the salinity of the water-based sample, the optical detection system between a marine analysis mode for analysing water-based samples having a salinity level within a first threshold value range, where in the marine analysis mode, the second optical analyser module is activated such that the water-based sample exiting the first optical analyser module is directed for further analysis to the second optical analyser module, and a freshwater analysis mode for analysing water-based samples having a salinity level within a second threshold value range, where in the second analysis mode the second optical analyser is deactivated and the water-based sample exiting the first optical analyser module is directed to waste.

It has been found that the portable in situ Ion Chromatography system of the present invention is capable of dynamically adjusting the operation of the optical detection system to facilitate the detection of inorganic analytes in water-based samples having different levels of salinity. Depending on the salinity level of the water-based sample, the processing unit is configured to switch the fluidic configuration and optical detection system between a marine analysis mode, also referred to as a two-dimensional (2D) analysis, and a freshwater mode, also referred to as a one-dimensional (1D) analysis, for the respective detection of inorganic analytes in water samples having a salinity level within a first threshold value range and a second threshold value range. For example, the marine analysis mode may be activated to monitor water-based samples obtained from estuaries, oceans, or from other types of water environments with salt content over a predetermined threshold, e.g. water-based samples having a salt content above 0.5 ppt (parts per thousand) up to full salinity e.g. 32 ppt. Similarly, the freshwater mode may be activated to detect inorganic analytes from water-based samples obtained from the freshwater environment such as lakes, rivers, and in general from another type of water environments having a salt content within a second predetermined threshold, e.g. water-based samples having salt content between 0 and 0.5 ppt. During the marine analysis mode, the water-based sample is processed first by the first optical analyser module and then by the second optical analyser module. In the freshwater mode, the water-based sample is only processed by the first optical analyser mode. In general, the second optical analyser module is selectively activated to analyse inorganic analytes in water-based samples having a salinity level within the first threshold value, as indicated above. Contrary to the existing solution, the monitoring device offers a means for dynamic, versatile and adaptable detection of inorganic analytes in a range of natural waters and waters of varying salinity. The present approach enables superior selectivity and sensitivity of inorganic analytes when covering a broader spectrum of water samples of varying salinity. Furthermore, the optical detection system is encased in a housing to ensure its direct operation in the desired monitoring location. The housing defines an internal space for housing the optical detection system, which is removably covered by a cover. The cover is positioned in an open position to expose the internal space and provide access to the optical detection system and a closed position to cover the internal space. A housing may be adapted to be weatherproof and/or shockproof. For example, a watertight connection may be formed between the cover and the internal space when the cover is in the closed position, e.g. using a sealing member around the edges of the internal space and/or cover. Furthermore, shock-absorbing means may be incorporated in the housing to prevent damage from accidental drops that could damage the optical detection system. For example, the housing may be made from a shock-absorbing material, e.g. plastic casing, and the like. In general, the system of the present invention is adapted to be portable and easily transportable to the desired location. The system of the present invention is provided with a power source, thereby ensuring its independent operation at the desired location. The power source may be a battery, which may be further coupled to power generating means such as solar panels, wind turbines, and the like.

According to embodiments of the present invention, the processing unit is configured to operate the optical detection system between the marine and freshwater mode based on information obtained from one or more salinity-type sensors of the optical detection system.

According to embodiments of the present invention, the processing unit is configured to operate the optical detection system between the first and second analysis mode based information extracted from a chromatogram generated by the processing unit from the information received from the first analyser during the analysis of the water-based sample.

According to embodiments of the present invention, the chromatogram information comprises any one of or a combination of peak retention times, injection peak height, retention time, baseline drift, and peak resolution (Rs). The processing unit is configured to determine the peak resolution (Rs) by calculating the separation distance between an injection peak and the next immediate peak in the chromatogram.

The optical detection system of the present invention may be switched between marine analysis mode and freshwater mode by the processing unit. For example, the processing unit may switch the operation of the optical detection system based on a user trigger signal, e.g. generated by a user operating a switch indicating the desired analysis mode. Similarly, the trigger signal may be received at the processing unit via a communication module, e.g. via a wirelessly communicated signal.

Furthermore, the optical detection system may comprise a sensor module that is communicatively coupled to the processing module. The sensor module may comprise one or more commercially available salinity type sensors that are configured to measure the salinity of the water-based sample. The processing unit may monitor the information obtained from one or more salinity type sensors and accordingly switch the operation of the optical detection system to the desired analysis mode.

Moreover, the processing unit may be configured to operate the optical detection system based on chromatogram feature information extracted from a chromatogram generated from information received from the first optical analyser during the analysis of the water-based sample. For example, the processing unit may determine, based on the analysis of the chromatogram generated from the first optical analyser that the water-based sample is from a marine environment or saline in nature rather than a freshwater environment and accordingly dynamically switch the operation of the optical detection system from a freshwater analysis mode to marine analysis mode. The chromatogram features used to determine whether the water-based sample is from a freshwater or marine environment, may include, but are not limited, to the peak retention time between injection and elutuon of the water-based sample, injection peak height, peak resolution (Rs) between two consecutive peaks e.g. injection peak and next peak peak width baseline drift, and the like. The processing unit is configured to monitor the values associated with the chromatiogram features mentioned above, and when the value of one or more features is within a threshold range, indicative of saline water-based sample, to switch the operation of the optical detection system to the marine analysis mode.

In this way, the system of the present invention may dynamically and, without user intervention, switch between the freshwater and marine analysis mode. Therefore, the portable Ion Chromatography system may be used in aquatic environments with varying and/or fluctuating salinity levels, e.g. estuaries, and the like.

According to embodiments of the present invention, each of the first and the second optical analyser modules comprises:

a guard column configured to separate anions in the water-based sample; and

an optical detection cell configured to analyse the water-based sample exiting the guard column for the detection of inorganic analytes.

According to embodiments of the present invention, the optical detection cell comprises an Ultra-Violet (UV) Light Emitting Diode, LED emitting light at wavelengths between 235 nm and 255 nm. The use of UV LEDs has been found to increase the longevity of the optical detection cell, lower the power consumption, and increase measurement sensitivity.

It has been found that with the present invention, the optical detector combined with a guard column configured for separating anions in the water-based sample achieves effective anion exchange separation. In particular, when operating the sample analysis system in the second operation mode, the water-based sample is processed by the guard column of each of the first and the second analyser modules. Through the application of the two-dimensional (2D) analysis, interferences and coelution associated with the high anion concentrations present within the seawater matrix are eliminated, and the selective detection of inorganic analytes, such as nitrate, is achieved. This approach allows maximising chromatographic separation in conditions where the water-based sample is difficult to resolve because different chemical compounds, such as major ion constituent, elute from the guard column at the same time, making separation and identification difficult. The present Ion Chromatography system incorporates two separate stages that operate in a manner that enabled either direct freshwater analysis or rapid matrix elimination for marine analysis in a single portable package.

According to embodiments of the present invention, the optical detection system comprises an eluent delivery system configured to direct a predetermined volume of eluent from an eluent source to the first and/or second optical detection system.

According to embodiments of the present invention, the eluent is sodium chloride, NaCl, eluent. The eluent has been selected to facilitate on-column matrix elimination using two guard columns, e.g. AG15. The use of NaCl eluent was successful, highlighting an environmentally friendly eluent and analyser. It has been found that flushing of the selected eluent into the guard column provides on-column matrix elimination, which mitigates the matrix effects of difficult to resolve samples such as seawater. The major matrix ion of the eluent dissolves molecules absorbed to the surface of the column in order to maximise chromatographic separation in conditions where the water-based sample is difficult to resolve. The portable in situ Ion Chromatography system may employ an on-column matrix elimination using sodium chloride (NaCl) eluent in which the chloride matrix ion in the eluent overcame the matrix effect of saline samples and allowed for the detection of various anions, such as nitrate, using direct UV detection. As such, the portable in situ Ion Chromatography system utilises an automated 1D/2D analysis method, in combination with on-column matrix elimination using sodium chloride eluent, for the detection of nitrate in freshwater and seawater matrix.

According to embodiments of the present invention, the optical detection system comprises a sample delivery system configured to direct a predetermined volume of the water-based sample from a sample reservoir to the first analyser module.

According to embodiments of the present invention, the optical detection system comprises a first and a second injection valve configured to direct, depending on the analysis mode, the water-based sample and eluent respectively to a sample loop of the first and second optical analyser.

The sample delivery and eluent delivery systems are operated according to the analysis mode, thereby ensuring that the sample and the eluent are directed to the desired optical detection analyser module.

According to embodiments of the present invention, the optical detection system comprises:

a sample reservoir configured to store the water-based sample;

a sample pump module configured to pump from a water-based source a predetermined volume of water-based sample to the sample reservoir;

at least one waste container configured to store the water-based sample exiting the first and/or second optical analyser module; and

an electric power source configured to power the optical detection system.

The sample pump module may be operated by the processing unit to draw water sample directly from the water source, which is then directed to a sample reservoir. The sample pump may be operated at a predetermined interval based on an analysis schedule stored in the processing unit.

According to embodiments of the present invention, the optical detection system comprises a communication module configured to communicate information between the processing unit and a remote monitoring platform.

The Ion Chromatography system of the present invention is configured to exchange information with a user monitoring platform. For example, the processing unit may communicate the information received from the first and second optical analysers to a user monitoring platform, where the information is displayed on a user interface of the user monitoring platform. The user may further control the operation of the Ion Chromatography system through the user monitoring platform, e.g. issuing commands to the processing unit. The processing unit may be configured to further monitor a range of parameters associate with the optical detection system, which are communicated to the user monitoring platform. For example, the set of parameters may include but are not limited to monitoring the eluent level in the eluent storage, the energy levels of the power source, the operation of components, reporting malfunctions, and the like.

According to embodiments of the present invention, the system is used for detecting inorganic analytes in water-based samples of different salinity levels. For example, the portable in situ Ion Chromatography of the present invention may be used to detect nitrite, nitrate, iodide, iodate in freshwater sample, and at least nitrate in saline water-based samples.

According to a third aspect of the present invention, a method for detecting inorganic analytes in water-based samples of different salinity levels using a portable in situ Ion Chromatography system of the first aspect is provided. The method comprising:

receiving a water-based sample at an optical detection system of the portable in situ Ion chromatography system;

directing the water-based sample to a first optical analyser module for the optical detection system;

determining, at a processing unit, based on information received from the first optical analyser the salinity level of the water-based sample;

wherein if the salinity level is within a first threshold value, the processing unit is configured to switch the operation of the optical detection system to a marine analysis mode, which marine analysis model comprises the steps of:

activating a second optical analyser to process the water-based sample exiting the first analyser; and

processing the information received from the second optical analyser to determine the levels of inorganic analytes.

## List of Figures

Preferred embodiment of the present invention will now be described, by way of example only, with reference to the accompanying drawings, in which:

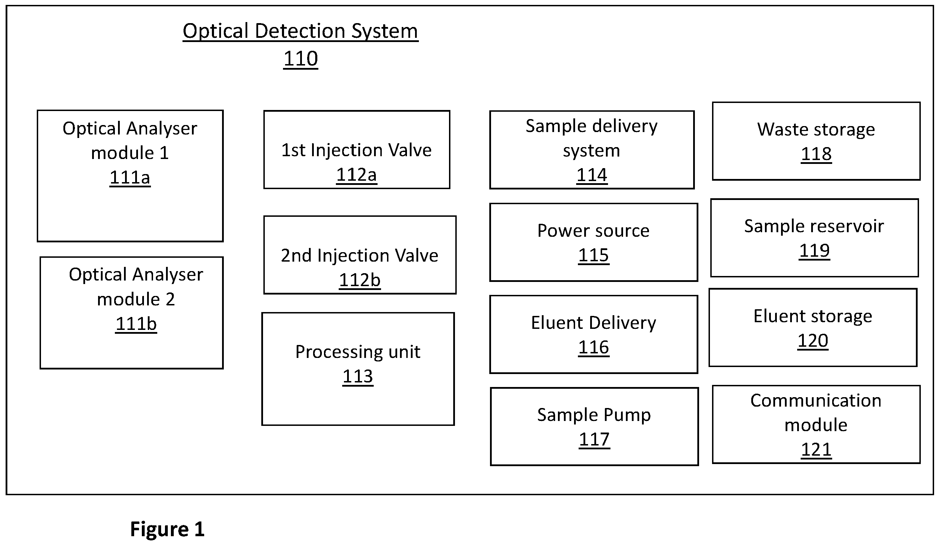

Figures 1 shows an exemplified configuration of the portable in-situ Ion Chromatography system according to embodiments of the present invention.

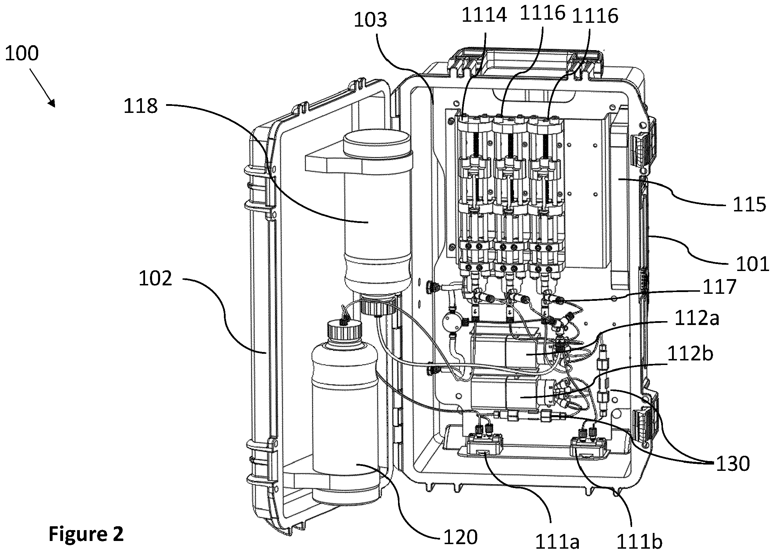

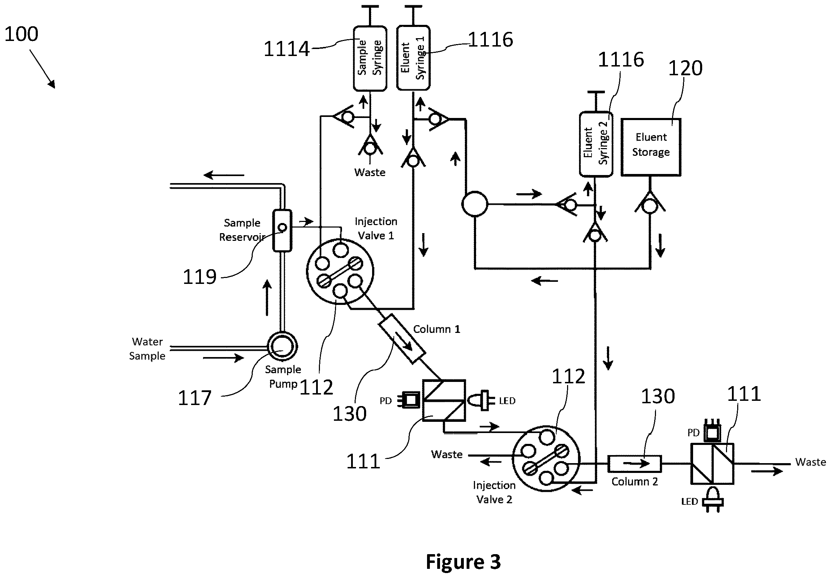

Figure 2 shows a graphical representation of the portable in-situ Ion chromatography system shown in Figure 3 according to embodiments of the present invention.

Figure 3 shows an exemplified fluidic schematic of the portable in-situ Ion Chromatography system shown in Figure 1 and 2 according to embodiments of the present invention.

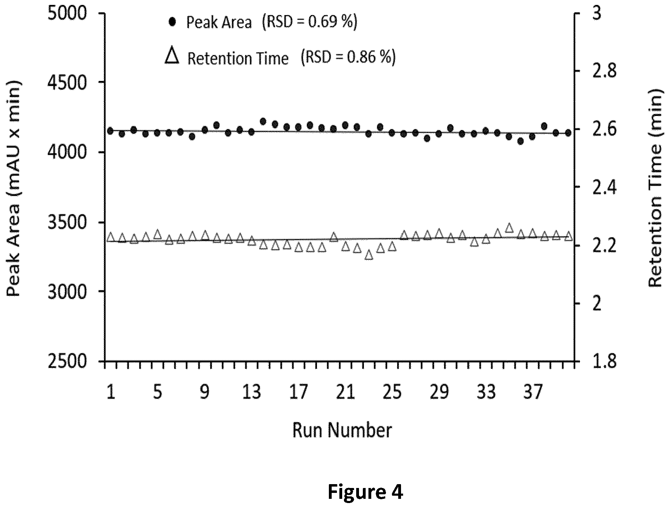

Figure 4 shows results obtained from the Ion Chromatography system of Figures 1-3 showing the repeatability of peak area and retention time values determined using the 2D IC configuration analysing 5 mg L$^{-1}$ NO$_3^-$ artificial seawater standard over 40 sequential runs and associated RSD values.

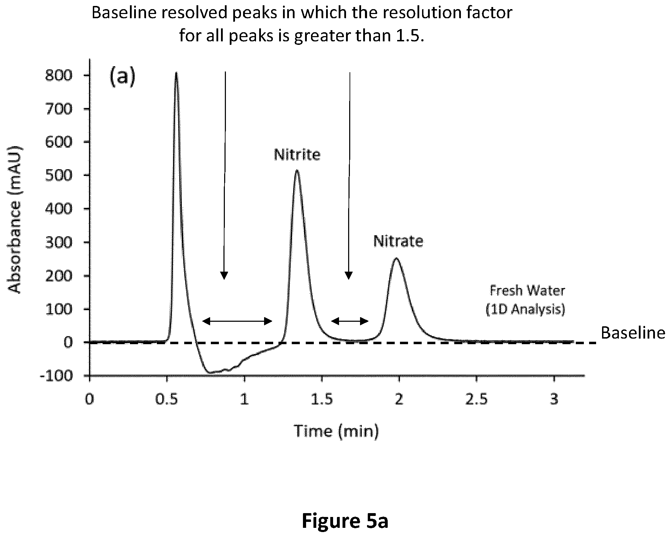

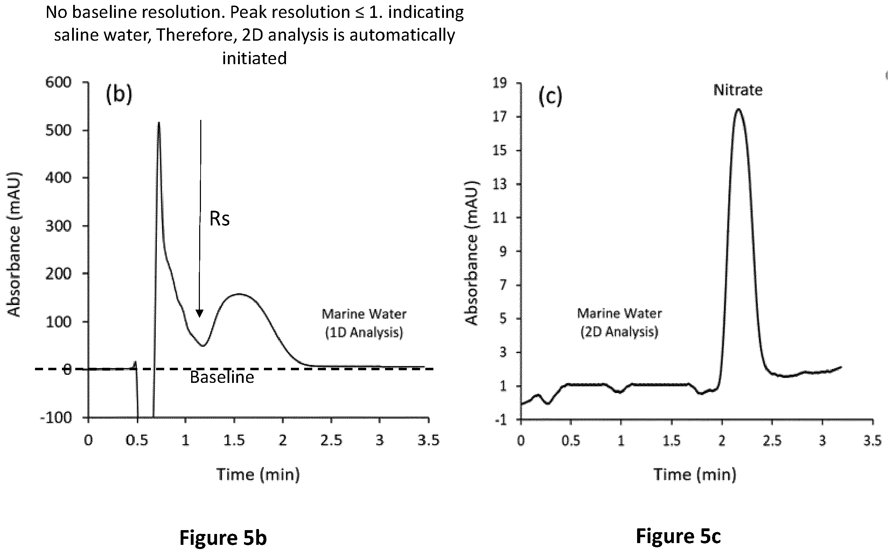

Figures 5a-5c show chromatogramss obtained from the Ion Chormatographjy system of Figure 1-3, figure 5a) shows a chromatogram of a 10 mg L-1 nitrite and 20 mg L-1 nitrate freshwater standard with a 10 mg L-1 mix of chloride, bromide, fluoride, sulphate and carbonate generated using 1D analysis. Eluent: 200 mM NaCl, flowrate: 0.7 mL min-1, 195 $\mu$L sample loop. Baseline resolved peaks are shown Figure 5b) shows a chromatogram of a 40 mg L-1 nitrate artificial seawater standard with 10 mg L-1 nitrite and iodate and 50 mg L-1 bromide using 1D analysis, highlighting coelution and ineffective detection of nitrate. A lack of baseline resolution and unresolved peaks are observed. Figure 5c) chromatogram of a 5 mg L-1 nitrate artificial seawater standard with 10 mg L-1 nitrite and iodate and 50 mg L-1 bromide using 2D analysis, highlighting selective and sensitive detection of nitrate.

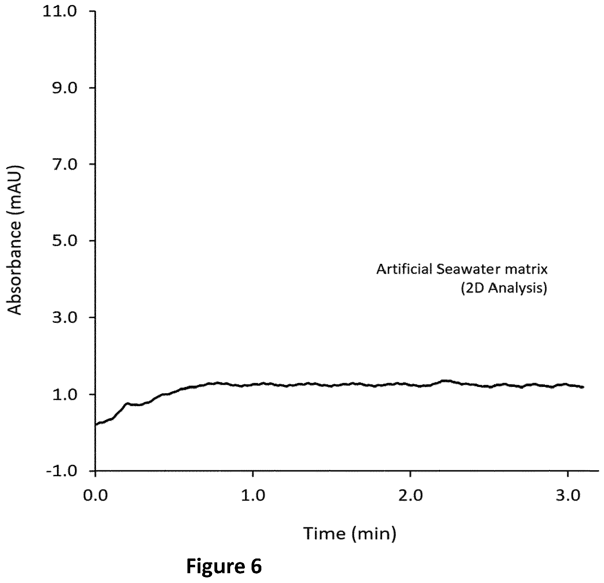

Figure 6 shows chromatogram of blank artificial seawater solution, without nitrate or nitrite present, using 2D ion chromatography configuration and 235 nm LED optical detector, highlighting the effective elimination of interfering anions from the sample through the application of the 2D IC approach. Eluent: 200 mM NaCl, flowrate: 0.7 mL min$^{-1}$, 195 $\mu$L sample loop.

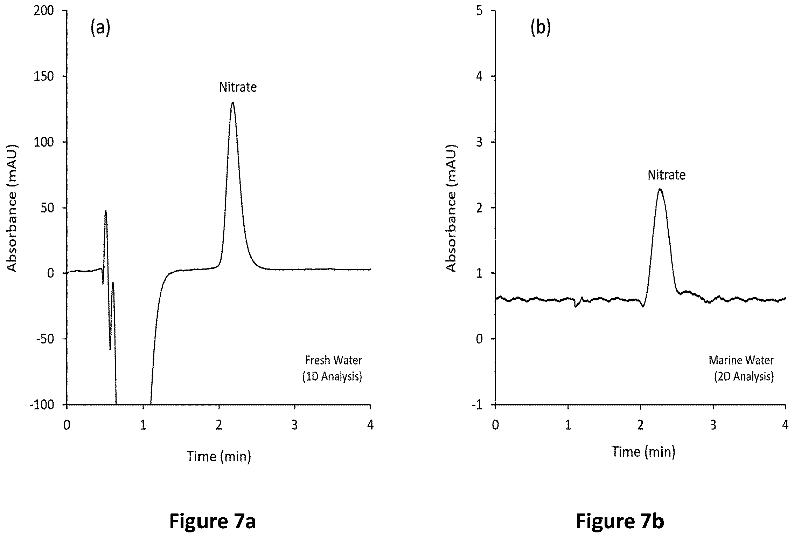

Figures 7a and 7b shows chromatograms generated from freshwater and marine water sample using the Ion Chromatography system of figure 1-3, figure 7a) shows a chromatogram of sample Freshwater A generated using 1D IC. Eluent: 200 mM NaCl, flowrate: 0.7 mL min-1, 195 $\mu$L sample loop with AG15 guard column for separation and 235 nm LED optical detection, figure 7b) Chromatogram of environmental seawater sample generated through 2D IC.

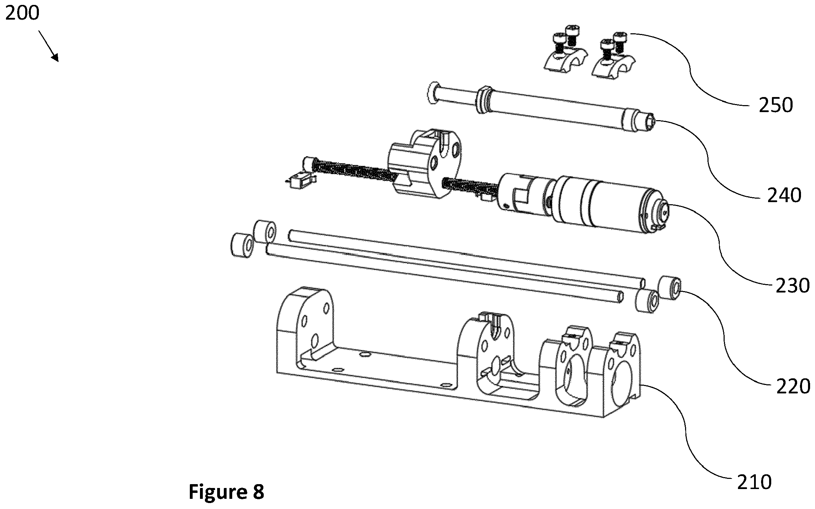

Figure 8 shows an exploded view of an exemplified 3D-printed syringe pump used within the portable analyser for sample intake and eluent delivery according to embodiments of the present invention.

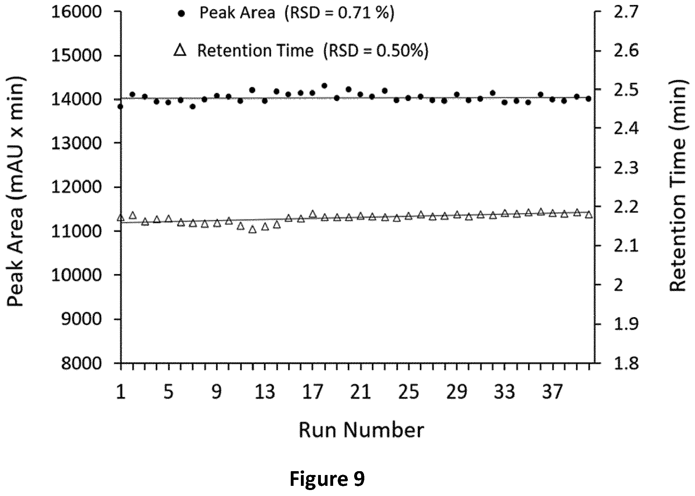

Figure 9 shows results for chromatographic repeatability of freshwater analysis using one-dimensional Ion Chromatography (IC). Repeatability of peak area and retention time values for nitrate determined using the one-dimensional configuration of the IC analyser, analysing 5 mg L-1 NO3- standard over 40 sequential runs and associated RSD values.

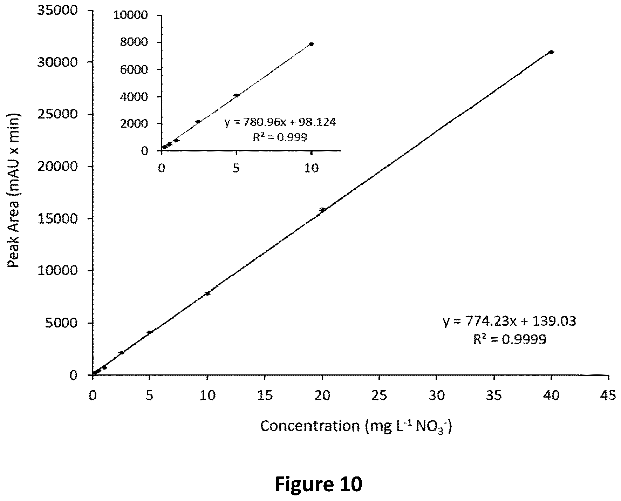

Figure 10 shows linearity and calibration plots for nitrate determination in marine water using the portable analyser shown in Figures 1-3. The linear range determined was 0.1 - 40 mg L-1 NO3- based on peak areas (error bars are standard deviations for n=3 replicates). Eluent: 200 mM NaCl, flowrate: 0.7 mL min-1, 195 $\mu$L sample loop using 2D ion chromatography analyser with two AG15 guard columns for separation and 235 nm LED optical detection.

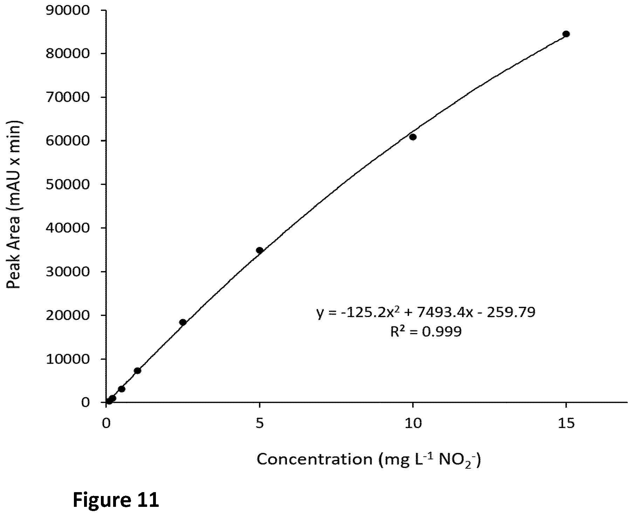

Figure 11 shows linearity and calibration plots generated for freshwater analysis using the portable analyser shown in Figures 1-3. The linear range determined was 0.1 - 15 mg L-1 NO2- based on peak areas (error bars are standard deviations for n=3 replicates). Eluent: 200 mM NaCl, flowrate: 0.7 mL min-1, 195 $\mu$L sample loop using 1D IC analyser with AG15 guard column for separation and 235 nm LED optical detection.

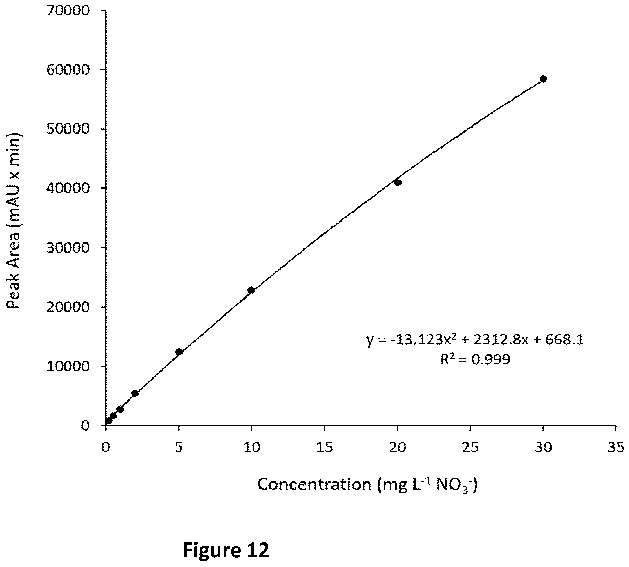

Figure 12 shows a calibration plot for nitrate determination in freshwater using the portable analyser shown in Figures 1-3. The linear range determined was 0.2 - 30 mg L-1 NO3- based on peak areas (error bars are standard deviations for n=3 replicates). Eluent: 200 mM NaCl, flowrate: 0.7 mL min-1, 195 $\mu$L sample loop using 1D IC analyser with AG15 guard column for separation and 235 nm LED optical detection.

## Detailed Description

Figure 1 shows an exemplified configuration of a portable situ Ion Chromatography system 100 according to embodiments of the present invention. System 100 is configured to perform *in-situ* analysis of water-based samples obtained from an aquatic environment, e.g. rivers, lakes, sea, water reservoirs, and the like. The system 100 may be configured to repeatedly analyse a fluid sample at user-configurable time frames to determine whether the analytes in the fluid samples are within the prescribed limits. System 100 is configured to analyse water-based samples of different salinity levels. For example, system 100 is configured to analyse water-based sample obtained from freshwater aquatic environments, having a salinity level between 0.0 to 0.5 parts per thousand (ppt) of salt content, and marine aquatic environment, having a salinity level above 0.5 ppt of salt content.

System 100 comprises an optical detection system 110 comprising a first optical analyser module 11a and a second optical analyser module 111b, which are fluidly connected in a cascading configuration via a second injection valve 112b. The optical detection system 110 is configured to operate in either a freshwater analysis mode or a marine analysis mode. In the freshwater analysis mode, the water-based sample is analysed only by the first optical analyser module 111a and the second injection valve 112b is configured to direct the sample exiting the first optical detection analyser 111a to waster storage 119, thereby by-passing the second optical analyser module 111b. In the marine analysis mode, the second injection valve 112b is configured to direct the water-based sample exiting the first optical analyser module 111a to the second optical analyser module 112b. The operation of the optical detection system 110 is controlled by a processing unit 113, which is configured to receive information obtained from the first and second optical analysers to determine the corresponding level of inorganic analytes in the corresponding freshwater or marine water-based sample. The processing unit 113 may be configured to determine the type of water-based sample, freshwater or marine, from information obtained from the analysis of the water-based sample by the first optical analyser module 111a. For example, the processing unit 113 may analyse the measurement values obtained from the analysis and accordingly determine the type of water-based sample, e.g. by analysing certain features from the chromatogram obtained such as peak retention time, baseline drift, and the like.

The processing unit 113 may further determine the type of the water-based sample based on information received from one or more salinity type sensors. The sensors may be configured to monitor any of the major ion constituents of the water-based sample, including Chloride (Cl), Sodium (Na+), Sulfate (SO42-), Magnesium (Mg2+), Calcium (Ca2+), Potassium (K+), Bicarbonate(HCO3-), Strontium (Sr2+), Bromide (Br-), and/or Borate (BO33-). The level of salinity in the water-based sample may be the result of a combination of any of the monitored constituent of the water-based sample.

The optical detection system 110 is provided with a sample delivery system 114 configured to deliver via the first injection valve 111, a predetermined volume of the water-based sample from a sample reservoir 119 to the first optical analyser module 111a. The water-based sample is obtained from the desired aquatic environment via a sample pump module 117, which is configured to direct a volume of the water-based sample to the sample reservoir 119. An eluent delivery system 116 is configured to deliver a predetermined volume of eluent from the eluent storage 120 to the first optical analyser module 111a via the first injection valve 112a and to the second optical analyser via the second injection valve 112b.

System 100 may be provided with a power source 150, e.g. a battery, solar panel operated battery and the like configured to provide the necessary power to each module of the Ion Chromatography system 100.

Furthermore, a range of sensors may be deployed to determine different environmental and system parameters such as syringe back-pressure, eluent volume, leakage, current, temperature, humidity, and the like. The processing unit 113 may be configured to process and transmit the data collected from the sensors and the first and second optical analysers 111a and 111b to a remote monitoring platform via a communication module 121. The remote monitoring platform may be configured to monitor and control the operation of a plurality of portable in-situ Ion Chromatography systems 100 deployed in different locations.

## Exemplified Portable Analyser

Figure 2 shows an exemplified representation of the portable in-situ Ion Chromatography system 100 according to embodiments of the present invention. As previously discussed with reference to figure 1, the analyser 100 incorporated two separation stages that operated in a manner that enabled either direct freshwater analysis or rapid matrix elimination for marine analysis in a single portable package. Anion exchange separation was achieved through the use of two 4 x 50 mm Dionex AG15 guard columns 130 from Thermo Fisher Scientific (Sunnyvale, CA). The twin eluent syringe pumps 1116 configuration of the eluent delivery system 116 were each coupled to one of two automated 6 port 2-position injection valves 112a and 112b provided by VICI AG (Schenkon, Switzerland). Optical detection was achieved using the first and the second optical analysers 111a and 111b, each comprising a low-cost optical detection cell, as disclosed in WO2019228686A1, employing two 235 nm deep UV-LEDs provided by Crystal IS (Green Island, NY, USA) and two UVC photodiodes (TOCON_C1) with integrated amplifiers purchased from Sglux GmbH (Berlin, Germany). The processing unit 113, which is in the form of an embedded system, consisted of an ARM-based microcontroller (Teensy 3.6) which controlled all the timing and actuation of the syringe pumps 116 and 6-way valves 112a and 112b as well as the sample intake pump 117 and a sample intake syringe pump 1114, which is part of the sample delivery system 114. Dedicated motor controllers (TB6612FNG) ensured the syringe pumps ran smoothly while simple on-off signals enabled the sample intake pump 117 and 6-way valve operation. The deep UV LED intensity for each optical detection cell was controlled through a constant current driver (AL8805) for stable operation. UVC photodiode signals were sampled using a 16-bit analogue to digital converter (ADS1115). Separation data processed by the microcontroller was stored on an SD card for easy retrieval. A communication module may be further provided so that the separation data can be communicated directly to a remote monitoring platform. Electronic components were sourced from Mouser Electronics (Texas, USA). Power to the system was supplied from a Lithium-Polymer battery pack (Voltaic V88) purchased from Voltaic Systems. The system 100 further is provided with a housing 101 defining an internal space 103 for housing the different components of the system 100, and a cover 102 configured to removably cover the internal space 103. The housing 101 may be made from a reinforced and lightweight material, e.g. metal, reinforced plastic, and the like, capable of protecting the components housed in the internal space 103 of the housing while ensuring portability and ease of transport of the system 100. Cover 102, when closed, may watertightly seal the internal space, e.g. by means of a sealing member provided on the cover 102, thereby ensuring the longevity of the portable system 100. In general, the housing 101 in the form of a casing configured, when closed, to watertightly seal the internal space 102.

Exemplified Analyser operation

Figure 3 shows an exemplified fluidic schematic of the analyser 100, representing the systems operational flow. Three 3D printed syringe pumps 1114 and 1116, comparable to those described within the portable IC analyser developed by Murray et al. (2020), were utilised within the system. The design and configuration of thesyringe pumps 1114 and 1116 is illustrated in figure 8. The syringe pump assembly 200 shown in figure 8 comrpises a 3D printed pump base 210, a drive plate guide rails 220 configured to be coupled to pump base 210, a syringe drive system 230 configured to be secured on the pump base 210 and cooperating with the drive plate guide rails 220, a 2.5 mL glass syringe 240 mounted on the pump base 210 and configured to cooperate with the guide rails 220 and drive system 230, and a pair of syring clamps 250 for securing the assembly. The 2.5 mL syringe pumps 1114 and 1116 were responsible for eluent delivery, and one 1 mL syringe pump 117 was used for drawing a sample from the sample reservoir 119 through the sample loop (195 μL), and hence filling the loop. During stage 1 of the analyser operation for marine analysis, the sample syringe 1114 and the eluent syringes 1116 operated in unison, with the sample syringe 1114 drawing in the sample and filling the sample loop, and the eluent syringes 1116 drawing in 2.5 ml of eluent each from the eluent storage 120. Once these syringes were full, parallel flushing of the sample syringe and eluent syringe 1 began, with the sample syringe 1114 flushing to waste and eluent syringe 1 1116 directing the flow of eluent through the first six-way valve 112a to column 1 130 and detector 1 111a, at a flow rate of 0.7 mL min-1.

As eluent syringe 1 1116 began flushing (time = 0 s), the first six-way valve 112a remained in the load position until 24 seconds had passed, after which the valve automatically switched to the inject position, injecting the sample. Eluent syringe 1 1116 continued to empty until it reached its home position, defined by the activation of limit switches.

During stage 2 of analyser operation for marine analysis, the flow from detector 1 111a was directed to waste through the second six-way valve 112b until 118 seconds had passed since the beginning of eluent syringe 1 1116 flushing. After this time, the second six-way valve 112b automatically switched to the load position, loading the second sample loop (800 μL). This timing corresponded to the start of the elution of the nitrate band from column 1 130. Eluent syringe 2 1116 began flushing after 177 seconds had passed, directing flow through the second six-way valve 112b to column 2 130 and detector 2 111b. The second six-way valve 112b automatically switched to inject after 207 seconds had passed, corresponding to the end of the elution of the nitrate band. The valve 112b remained in the inject position for 17 seconds before automatically switching back to the load position. The 17 seconds injection time corresponds to a sample injection volume of 198 μL. The flow continued from the valve 112b to column 2 130 and detector 2 111b and finally to a waste container 118 until eluent syringe 2 1116 reached its home position.

When the analyser 100 was in freshwater operational mode, the second stage of the operation was by-passed, and stage one directly analysed the sample using eluent from eluent syringe 1 1116 while data acquisition by the processing unit 113 was obtained from column 1 130 and detector 1 111a only. Flow from detector 1 111a was directed to waste 118 through the second six-way valve 112a only, and the second stage was not activated.

Experimental Results

Chromatographic Repeatability

The repeatability of the marine analysis system employing the 2D IC configuration was assessed by analysing 40 sequential runs (n=40) of a 5 mg L-1 NO3- artificial seawater standard. The RSD values for both peak area and retention times were below 1 %, and the repeatability which was achieved is graphically depicted in figure 4. The peak width which was observed for nitrate during this analysis was 0.51 mins, and the associated asymmetry factor (As) was 1.4. The repeatability demonstrated when analysing freshwater 5 mg L-1 NO3- standard using the direct 1D IC configuration is graphically presented in Figure 9. The RSD values for both peak area and retention times when performing the freshwater analysis (n=40) were 0.71 % and 0.50 %, respectively.

Assessment of Interfering Anions

Anions commonly found in both fresh water and seawater were investigated to ensure no coelution was occurring when detecting nitrate. An anion mixture containing 20 mg $L^{-1}$ nitrate and 10 mg $L^{-1}$ nitrite, fluoride, bromide, chloride, iodide, sulphate and carbonate was analysed directly by the analyser 100 through 1D IC. The chromatogram which was generated is depicted in Figure 5 (a), highlighting selective detection of both nitrite and nitrate when analysing a freshwater matrix. This is the chromatogram type observed when analysing a freshwater matrix. As can be seen, all peaks are resolved. Peak resolution, meaning the separation between two peaks, can be quantified using the resolution factor (R) which is obtained from the chromatogram. It is determined by subtracting the retention time of peak A from peak B then dividing this by the sum of the two peak widths. This total value is then multiplied by 2 to obtain the R value. When analysing a freshwater matrix using 1D analysis, baseline resolved peaks are observed with R values of > 1.5, as shown in Figure 5 (a).

A 40 mg $L^{-1}$ nitrate artificial seawater (15210 mg $L^{-1}$ chloride, 2728 mg $L^{-1}$ sulphate, 145 mg $L^{-1}$ bicarbonate) standard with 10 mg $L^{-1}$ nitrite and iodate and 50 mg $L^{-1}$ bromide was analysed using direct 1D analysis, the corresponding chromatogram is shown in Figure 5 (b). As can be seen, severe coelution was observed, and selective nitrate detection was not achieved. In contrast to the freshwater matrix, no baseline resolution is observed and peak resolution is significantly compromised. R values of $\leq 1$ are observed. When these values are experienced, saline water matrix is indicated and the processing unit of the system automatically initiates 2D analysis. A 5 mg $L^{-1}$ nitrate artificial seawater standard with 10 mg $L^{-1}$ nitrite and iodate and 50 mg $L^{-1}$ bromide was analysed employing the 2D IC analysis configuration. The generated chromatogram is shown in Figure 5 (c). Through the application of the 2D IC, interferences and coelution associated with the high anion concentrations present within the seawater matrix are eliminated, and selective detection of nitrate was demonstrated. A chromatogram for the artificial seawater matrix, without nitrite or nitrate present, following 2D IC is shown in Figure 6 which highlights the effectiveness of the 2D configuration in eliminating the interfering effects of the highly saline matrix.

Analytical Performance

The detection of nitrate within marine water matrices was achieved in under 3 minutes using 2D IC employing isocratic elution using 200 mM NaCl eluent at a flow rate of 0.7 mL min$^{-1}$ through two AG15 columns 130. A sample injection of 195 $\mu$L was utilised in combination with two 235 nm LED-based optical UV detectors. The linear calibration ranges and associated $R^2$ values for nitrite and nitrate are presented in Table 1. The corresponding limit of detection (LOD) values, estimated using a signal-noise-ratio (S/N) = 3, are also shown within the table. The nitrate calibration curve for marine analysis is illustrated in Figure 10. Similarly, the nitrite and nitrate calibration curves for freshwater analysis are illustrated in Figures 11 and 12. Analytical ranges for freshwater analysis were achieved using calibration curves based on peak area values and a polynomial fit. Detection of nitrite and nitrate in freshwater was achieved directly with 1D IC. A single 2.5 mL eluent syringe pump using 200 mM NaCl eluent with AG15 column 130, a sample injection of 195 $\mu$L in combination with a 235 nm optical UV detector successfully separated the two anions in under 2.5 min.

**Table 1:** Performance of portable analyser, analysing marine matrices through 2D IC analysis and freshwater matrices through direct 1D IC analysis.

| Analysis | Analyte | Linear Range | $R^2$ | LOD |
|---|---|---|---|---|
| Marine | Nitrate | 0.1 - 40 mg $L^{-1}$ $NO_3^-$ | 0.999 | 10 $\mu$g $L^{-1}$ $NO_3^-$ |
| Freshwater | Nitrite | 0.1 - 15 mg $L^{-1}$ $NO_2^-$ | 0.993 | 5 $\mu$g $L^{-1}$ $NO_2^-$ |
| | Nitrate | 0.2 - 30 mg $L^{-1}$ $NO_3^-$ | 0.997 | 20 $\mu$g $L^{-1}$ $NO_3^-$ |

Sample Analysis

A combination of blind standard solutions and environmental water samples were analysed. For freshwater analysis, two Environmental Protection Agency (EPA) intercalibration standards and one river water sample were provided by the Environmental Department within TelLab (Carlow, Ireland). Nitrate concentrations determined within the freshwater samples using the portable Ion Chromatography (IC) analyser 100, employing 1D analysis, were compared to those generated by an accredited Ion Chromatography (IC), and the determined concentrations with relative errors are shown in Table 2. The chromatogram generated for the analysis of sample 'Freshwater A' is depicted in Figure 7 (a). For marine analysis, blind artificial seawater standards were analysed using the portable IC analyser, employing 2D analysis. The determined nitrate concentrations with associated relative errors are also reported in Table 2.

**Table 2:** Nitrate concentrations determined within environmental freshwater samples and blind artificial seawater samples using multi-dimensional IC analyser ($n$=3)

| Sample | Analyte | Portable IC 1D Analysis (mg L$^{-1}$) | Accredited IC (mg L$^{-1}$) | Relative Error (%) |
|---|---|---|---|---|
| Freshwater A | $NO_3^-$ | 9.9 ± 0.08 | 9.5 ± 0.07 | 4.21 % |
| Freshwater B | $NO_3^-$ | < 0.1 | < 0.25 | - |
| Freshwater C | $NO_3^-$ | 11.4 ± 0.05 | 11.6 ± 0.09 | -1.72 % |
| | | | | |
| Sample | Analyte | Portable IC 2D Analysis (mg L$^{-1}$) | Standard Concentration (mg L$^{-1}$) | Relative Error (%) |
| Artificial Seawater A | $NO_3^-$ | 6.92 ± 0.08 | 7 | 1.14 % |
| Artificial Seawater B | $NO_3^-$ | 2.51 ± 0.03 | 2.5 | 0.4 % |

Using seawater sample taken from Tramore Bay, Co. Waterford, Ireland, recovery analysis was carried out according to the spiking and recovery procedure previously reported in E. Murray, E.P. Nesterenko, M. McCaul, A. Morrin, D. Diamond, B. Moore, A colorimetric method for use within portable test kits for nitrate determination in various water matrices, Anal. Methods, 9 (2017) 680-687. This seawater sample was spiked with 5 mg L$^{-1}$ NO$_3^-$ (Seawater A) and 7 mg L$^{-1}$ NO$_3^-$ (Seawater B). The calculation used to determine recovery is highlighted below.

Recovery = spiked sample conc. - ((unspiked sample conc. / 100 mL) x 99.5 mL)

Recovery % = recovery / spike concentration x 100

The nitrate concentration present in the neat seawater sample was first determined using the portable IC through 2D analysis, the chromatogram is shown in Figure 7 (b). The nitrate concentration was determined to be 0.37 mg L$^{-1}$ NO$_3^-$. This concentration was divided by 100 mL and then multiplied by 99.5 mL to account for the dilution caused by adding 0.5 mL of 1000 mg L$^{-1}$ NO$_3^-$ standard to make up 100 mL total volume in the seawater matrix. This final value was then subtracted from the spiked sample concentration to determine the recovery, which could then be expressed as a percentage. The nitrate concentrations which were determined for the spiked seawater samples and corresponding recovery values are shown in Table 3.

**Table 3:** Concentrations determined within artificial seawater blind standards and spiked environmental seawater samples using multi-dimensional IC analyser ($n$=3)

| Sample | Analyte | Portable IC 2D Analysis (mg L$^{-1}$) | Spike Concentration (mg L$^{-1}$) | Average Recovery (%) |
|---|---|---|---|---|
| Seawater A | $NO_3^-$ | 5.2 ± 0.08 | 5 | 104.37 % |
| Seawater B | $NO_3^-$ | 7.4 ± 0.04 | 7 | 106.07 % |

According to the present invention, a portable and fully automated Ion Chromatography (IC) system 100 is developed for the detection of nitrate in marine and natural (freshwater) waters. The system allowed for 1D or 2D Ion Chromatography (IC) analysis depending on the sample matrix to be analysed. For marine analysis, the developed method incorporated a 2D IC approach in combination with on-column matrix elimination techniques using two AG15 guard columns, a 200 mM NaCl eluent and detection with a low-cost 235 nm LED-based absorbance detector. Direct 1D analysis was implemented for monitoring nitrite and nitrate in freshwater samples. The use of NaCl eluent was successful, highlighting an environmentally friendly eluent and analyser.

Chromatographic repeatability was demonstrated, and an assessment of the effect of interfering ions was investigated. High levels of sensitivity and selectivity were shown for both the marine and freshwater analysis. Linear ranges applicable to environmental water monitoring were obtained for nitrate in marine water and for both nitrite and nitrate in freshwater. Recovery analysis was successfully carried out on seawater samples from Tramore, Co. Waterford, Ireland. The system proved successful in returning accurate and repeatable results for both marine and freshwater samples, showing its diverse application in the monitoring of a wide range of environmental waters.

Chemicals and Reagents used in the experiments

All chemicals used in the above experiments were of analytical grade. All solutions were prepared using high purity deionised water (Milli-Q). Sodium chloride eluent was prepared using a 5 M NaCl stock solution (Sigma Aldrich). Nitrate and nitrite stock solutions were prepared using $NaNO_3$ (Sigma Aldrich) and $NaNO_2$ (Sigma Aldrich), respectively. Artificial seawater was prepared according to the procedure set out by Metrohm in IC Application Note U-62, as described in Metrohm AG, IC Application Note U-62, Ammonium, nitrite, and nitrate in artificial seawater applying UV/VIS detection, Metrohm, Herisau, 2013. The following quantities of salts were added to 1 L of deionised water: 28 g NaCl, 7 g $MgSO_4.7H_2O$, 5 g $MgCl_2.6H_2O$, 1.61 g $CaCl_2.2H_2O$ and 0.4 g $NaHCO_3$. For the interference studies, nitrate, nitrite, carbonate, chloride, iodate, sulphate, bromide and fluoride stock solutions were prepared using $NaNO_3$ (Sigma Aldrich), $NaNO_2$ (Sigma Aldrich), $Na_2CO_3$ (Sigma Aldrich), NaCl (Fisher Chemicals), $KIO_3$ (PanReac AppliChem), $Na_2SO_4$ (Sigma Aldrich), KBr (Merck) and NaF (Sigma Aldrich), respectively. Working standards were prepared by diluting these stock solutions.

**Claims**

1. A portable in situ Ion Chromatography system for detecting inorganic analytes in water-based samples having different levels of salinity, the system comprising:

   a housing defining an internal space and comprising a cover configured to removably cover the internal space;
   an optical detection system disposed within the internal space and configured to receive and analyse awater-based sample for the detection of inorganic analytes, the optical detection system comprising

   a first optical analyser module configured to receive and analyse the water-based sample;
   a second optical analyser module configured, when activated, to receive and analyse the water-based sample exiting the first optical analyser module; and
   a processing unit configured to determine the levels of inorganic analytes in the water-based sample based on information received from the first and/or second optical analysers;

   wherein the processing unit is configured to operate, based on the salinity of the water-based sample, the optical detection system between a marine analysis mode for analysing water-based samples having a salinity level within a first threshold value range, where in the marine analysis mode, the second optical analyser module is activated such that the water-based sample exiting the first optical analyser module is directed for further analysis to the second optical analyser module, and a freshwater analysis mode for analysing water-based samples having a salinity level within a second threshold value range, where in the second analysis mode the second optical analyser is deactivated and the water-based sample exiting the first optical analyser module is directed to waste.

2. The system of claim 1, wherein the processing unit is configured to operate the optical detection system between the marine and freshwater mode based on information obtained from one or more salinity-type sensors of the optical detection system.

3. The system of claim 1 or 2, wherein the processing unit is configured to operate the optical detection system between the first and second analysis mode based on information extracted from a chromatogram generated by the processing unit from the information received from the first analyser during the analysis of the water-based sample.

4. The system of claim 3 wherein the chromatogram information comprises any one of or a combination of peak retention times, injection peak height, retention time, baseline drift, and peak resolution (Rs).

5. The system of claim 4, wherein the processing unit is configured to determine the peak resolution (Rs) by calculating the separation distance between an injection peak and the next immediate peak in the chromatogram.

6. The system of any preceding claims, wherein each of the first and the second optical analyser modules comprises:

a guard column configured to separate anions in the water-based sample; and
an optical detection cell configured to analyse the water-based sample exiting the guard column for the detection of inorganic analytes.

7. The system of claim 6, wherein the optical detection cell comprises an Ultra-Violet (UV) Light Emitting Diode, LED emitting light at wavelengths between 235nm and 255nm.

8. The system of claim 6 or 7, wherein the optical detection system comprises an eluent delivery system configured to direct a predetermined volume of eluent from an eluent source to the first and second optical detection system.

9. The system of claim 8, wherein the eluent is a sodium chloride eluent.

10. The system of any one of claims 6 to 9, wherein the optical detection system comprises a sample delivery system configured to direct a predetermined volume of the water-based sample from a sample reservoir to the first analyser module.

11. The system of claim 10, wherein the optical detection system comprises a first and a second injection valve configured to direct the water-based sample and eluent respectively to a sample loop of the first and second optical analyser.

12. The system of any one of claims 6 to 11 , wherein the optical detection system comprises:

a sample reservoir configured to store the water-based sample;
a sample pump module configured to pump from a water-based source a predetermined volume of water-based sample to the sample reservoir;
at least one waste container configured to store the analysed water-based sample exiting the first and/or second optical analyser module; and
an electric power source configured to power at least the optical detection system.

13. The system of any one of the preceding claims, wherein the optical detection system comprises a communication module configured to communicate information between the processing unit and a remote central platform.

14. Use of the a portable in situ Ion Chromatography system of any one of claims 1 to 15 for detecting inorganic analytes in water-based samples of different salinity levels.

15. A method for detecting inorganic analytes in water-based samples of different salinity levels using a portable in situ Ion Chromatography system of any one of claims 1 to 13, the method comprising:

receiving a water-based sample at an optical detection system of the portable in situ Ion chromatography system;
directing the water-based sample to a first optical analyser module for the optical detection system;
determining, at a processing unit, based on information received from the first optical analyser the salinity level of the water-based sample;
wherein if the salinity level is within a first threshold value, the processing unit is configured to switch the operation of the optical detection system to a marine analysis mode, which marine analysis model comprises the steps of:

activating a second optical analyser to process the water-based sample exiting the first analyser; and
processing the information received from the second optical analyser to determine the levels of inorganic analytes.

**Figure 1**

Optical Detection System
110

Optical Analyser module 1
111a

Optical Analyser module 2
111b

1st Injection Valve
112a

2nd Injection Valve
112b

Processing unit
113

Sample delivery system
114

Power source
115

Eluent Delivery
116

Sample Pump
117

Waste storage
118

Sample reservoir
119

Eluent storage
120

Communication module
121

Figure 2

**Figure 3**

Figure 4

Figure 5a

No baseline resolution. Peak resolution ≤ 1. indicating saline water, Therefore, 2D analysis is automatically initiated

**Figure 5b**

(b)

Rs

Baseline

Marine Water (1D Analysis)

**Figure 5c**

(c)

Marine Water (2D Analysis)

Nitrate

**Figure 6**

**Figure 7a**

Absorbance (mAU) vs Time (min), (a), Nitrate, Fresh Water (1D Analysis)

**Figure 7b**

Absorbance (mAU) vs Time (min), (b), Nitrate, Marine Water (2D Analysis)

**Figure 8**

200

210

220

230

240

250

Figure 9

**Figure 10**

Figure 11

$$y = -125.2x^2 + 7493.4x - 259.79$$
$$R^2 = 0.999$$

**Figure 12**

y = -13.123x² + 2312.8x + 668.1
R² = 0.999

Peak Area (mAU x min)

Concentration (mg L⁻¹ NO₃⁻)

Europäisches
Patentamt

European
Patent Office

Office européen
des brevets

**EUROPEAN SEARCH REPORT**

Application Number

EP 21 17 6724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2019/228686 A1 (T E LABORATORIES LTD [IE]) 5 December 2019 (2019-12-05) * claims 1,13; figure 6 * * abstract * | 1-15 | INV. G01N30/96 G01N30/88 G01N33/18 |
| A,D | KAZUAKI ITO ET AL: "Determination of nitrite, nitrate, bromide, and iodide in seawater by ion chromatography with UV detection using dilauryldimethylammonium-coated monolithic ODS columns and sodium chloride as an eluent", ANALYTICAL AND BIOANALYTICAL CHEMISTRY, SPRINGER, BERLIN, DE, vol. 404, no. 8, 18 September 2012 (2012-09-18), pages 2513-2517, XP035127387, ISSN: 1618-2650, DOI: 10.1007/S00216-012-6405-9 * abstract * * page 2514, left-hand column, line 43 - right-hand column, line 23 * | 1-15 | ADD. G01N21/33 G01N30/74 H01L25/16 |
| A | ZHU YONG ET AL: "Development of analytical methods for ammonium determination in seawater over the last two decades", TRAC TRENDS IN ANALYTICAL CHEMISTRY, ELSEVIER, AMSTERDAM, NL, vol. 119, 16 August 2019 (2019-08-16), XP085854200, ISSN: 0165-9936, DOI: 10.1016/J.TRAC.2019.115627 [retrieved on 2019-08-16] * abstract * * page 12, left-hand column, lines 4-39 * | 1,14,15 | TECHNICAL FIELDS SEARCHED (IPC) G01N H01L |

-/--

The present search report has been drawn up for all claims

| Place of search | Date of completion of the search | Examiner |
|---|---|---|
| Munich | 29 October 2021 | Marembert, Vincent |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

**EUROPEAN SEARCH REPORT**

Application Number

EP 21 17 6724

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MA JIAN ET AL: "Speciation and detection of arsenic in aqueous samples: A review of recent progress in non-atomic spectrometric methods", ANALYTICA CHIMICA ACTA, ELSEVIER, AMSTERDAM, NL, vol. 831, 23 April 2014 (2014-04-23), pages 1-23, XP029030496, ISSN: 0003-2670, DOI: 10.1016/J.ACA.2014.04.029 * page 21, left-hand column, lines 24-29 * * table 2 * ----- | 1,14,15 | |

TECHNICAL FIELDS
SEARCHED      (IPC)

The present search report has been drawn up for all claims

| Place of search | Date of completion of the search | Examiner |
|---|---|---|
| Munich | 29 October 2021 | Marembert, Vincent |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 17 6724

This annex lists the patent family members relating to the patent documents cited in the above-mentioned European search report.
The members are as contained in the European Patent Office EDP file on
The European Patent Office is in no way liable for these particulars which are merely given for the purpose of information.

29-10-2021

| Patent document cited in search report | Publication date | Patent family member(s) | | Publication date |
|---|---|---|---|---|
| WO 2019228686 A1 | 05-12-2019 | CN | 112219106 A | 12-01-2021 |
| | | EP | 3794330 A1 | 24-03-2021 |
| | | JP | 2021525380 A | 24-09-2021 |
| | | US | 2021208060 A1 | 08-07-2021 |
| | | WO | 2019228686 A1 | 05-12-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019228686 A1

**Non-patent literature cited in the description**

- **HADDAD et al.** On-column matrix elimination of high levels of chloride and sulfate in non-suppressed ion chromatography. *J. Chromatogr. A.,* 1991, vol. 546, 221-228
- **ITO et al.** Determination of nitrite, nitrate, bromide, and iodide in seawater by ion chromatography with UV detection using dilauryldimethylammonium-coated monolithic ODS columns and sodium chloride as an eluent. *Analytical and Bioanalytical Chemistry,* 2012, vol. 404, 2513-2517
- **WANG et al.** Determination of low-level anions in seawater by ion chromatography with cycling-column-switching. *J. Chromatogr. A,* 2012, vol. 1265, 186-190
- **E. MURRAY ; E.P. NESTERENKO ; M. MCCAUL ; A. MORRIN ; D. DIAMOND ; B. MOORE.** A colorimetric method for use within portable test kits for nitrate determination in various water matrices. *Anal. Methods,* 2017, vol. 9, 680-687